# EUROPÄISCHE PATENTSCHRIFT

(11) **EP 0 871 865 B1**
(45) Veröffentlichungstag und Bekanntmachung des Hinweises auf die Patenterteilung: **19.02.2003**
(21) Anmeldenummer: 95942708.9
(22) Anmeldetag: 20.12.1995
(51) Int. Cl.: G01N 21/85, G01N 21/35

(54) **VORRICHTUNG ZUR MESSUNG DES PARTIALDRUCKES VON IN FLÜSSIGKEITEN GELÖSTEN GASEN**
DEVICE FOR MEASURING THE PARTIAL PRESSURE OF GASES DISSOLVED IN LIQUIDS
DISPOSITIF DE MESURE DE LA PRESSION PARTIELLE DE GAZ DISSOUS DANS DES LIQUIDES

(30) Priorität: 21.12.1994 DE 4445668
(43) Veröffentlichungstag der Anmeldung: 21.10.1998
(73) Patentinhaber: EUROFERM GESELLSCHAFT FÜR FERMENTATION UND MESSTECHNIK MBH, 13355 Berlin (DE)
(72) Erfinder: DIECKMANN, Michael, D-10961 Berlin (DE); BUCHHOLZ, Rainer, D-13347 Berlin (DE)
(74) Vertreter: Fitzner, Uwe, Dr.
(86) Internationale Anmeldenummer: EP9505050
(87) Internationale Veröffentlichungsnummer: WO96019723

(56) Entgegenhaltungen:
- EP-A- 0 253 559
- US-A- 4 041 932
- US-A- 4 201 222
- PROCEEDINGS SPIE:IN-PROCESS OPTICAL MEASUREMENTS, Bd. 1012, 1988, Seiten 58-65, XP002005844 G.BOISD ET AL.: "ASPECTS OF OPTICAL FIBERS AND SPECTROMETRIC SENSORS IN CHEMICAL PROCESS AND INDUSTRIAL ENVIRONMENTS"

## Beschreibung

Die vorliegende Erfindung betrifft eine neuartige Vorrichtung zur Messung des Gaspartialdrucks in flüssigen Medien.

Vor allem im Bereich der Fermentationstechnologie hat sich zunehmend die Notwendigkeit ergeben, die Messung von Gasen über die Bestimmung des Partialdruckes vorzunehmen. So sind für die Ermittlung des Sauerstoff- und des Kohlendioxid-Partialdruckes spezielle Sonden entwickelt worden. Ein weitverbreitetes Beispiel hierfür sind die sog. Severinghauselektroden. Diese Vorrichtungen arbeiten mit membranbedeckten Einstab-pH-Elektroden (DE-OS 25 08 637, Biotechnol. Bioeng. 22(1980), 2411-2416, Biotechnol. Bioeng. 23(1981), 461-466). Bei diesem System befindet sich zwischen der gasselektiven Membran und der pH-Elektrode eine Elektrolytlösung oder -paste. Das Meßprinzip beruht darauf, daß Kohlendioxid in wäßriger Lösung Kohlensäure bildet, die zu einem Bicarbonatanion und einem Proton dissoziiert. Dieser Vorgang bewirkt in der Elektrolytlösung eine pH-Werts-Änderung, die mittels der pH-Sonde gemessen wird. Der Nachteil dieses Meßprinzips ist die Tatsache, daß Kohlendioxid nicht direkt, sondern seine ionische Form gemessen wird. Da der Anteil der ionischen Form unter 0,1 % liegt, ist diese Methode nicht ausreichend genau. Abgesehen davon stören andere flüchtige saure oder basische Gase die pH-Werts-Messung. Des weiteren ist ein sehr hoher Wartungsaufwand erforderlich.

Ferner sind aus dem Stand der Technik pCO₂-Optoden bekannt. Auch hier handelt es sich um ein membranbedecktes Sensorsystem (SPIE Vol. 798 Fiber Optic Sensors II (1987) S. 249-252; Anal. Chim. Acta 160 (1984) S. 305-309; Proc. Int. Meeting on Chemical Sensors, Fukuoka, Japan, Elsevier, S. 609-619, 1983, Talanta 35(1988)2 S.109-112, Anal.Chem. 65(1993) S.331-337, Fresenius Z. Anal. Chem. 325(1986) S. 387-392). Bei den pH-Optoden werden pH-Indikatoren, die in Abhängigkeit von der Protonenkonzentration ihre Absorptions- oder Fluoreszenzeigenschaften ändern, als Indikatorphase verwendet (Anal.Chem. 52(1980) S.864-869, DE-OS 3 343 636 und 3 343 637, US-Pat. Appl. 855 384). Trennt man den Indikator mit einer gaspermeablen Membran von dem Meßgut ab, können nur Gase, beispielsweise Kohlendioxid, durch die Membran zur Indikatorphase dringen und dort eine pH-Werts-Änderung durch Hydrolyse verursachen. Solche Kohlendioxid-Optoden arbeiten analog zu den Severinghaus-Elektroden. Die Nachteile optischer pH- und damit pCO₂-Messungen liegen in dem sehr eingeschränkten analytischen Meßbereich und der lonenstärkeabhängigkeit. Einer breiten Anwendung der Optoden stehen daneben die bereits hinsichtlich der Severinghaus-Elektroden genannten Nachteile entgegen.

Es ist ferner bekannt, die CO₂-Konzentration in Flüssigkeiten mittels abgeschwächter Totalreflektion zu bestimmen (The Chemical Engineer 498 (1991) S. 18). In einer Durchflußmeßze'le für fluide Stoffe, beispielsweise Bier, ist senkrecht zur Strömungsrichtung ein Sapphir-ATR (Attenuated Total Reflectance)-Kristall angeordnet. Das Infrarotlicht, das an einer Seite dem Kristall zugeführt wird, durchläuft den Kristall und wird mehrmals total reflektiert. Bei jeder Reflektion gelangt die Strahlung mehrere um in die Probenflüssigkeit und wird durch vorhandenes Kohlendioxid abgeschwächt. Die Restlichtmenge am anderen Ende des Kristalls wird gemessen. Nachteil dieser Methode ist, daß keine Partialdrücke gemessen werden können. Andererseits können bei sich verändernden Fluiden durch die Änderung der Reflektionseigenschaften Verfälschungen der Ergebnisse auftreten.

Aus der deutschen Offenlegungsschrift 2435493 ist ein Differenzdruckmeßgerät für die Bestimmung von Kohlensäure bekannt. Dieses Gerät kann jedoch nur in strömenden Medien eingesetzt werden. Daher eignet es sich insbesondere nicht für die Verwendung in herkömmlichen Rühr- oder Festmittelreaktoren, wie sie insbesondere in der Fermentationsindustrie zum Einsatz kommen.

Aus der deutschen Offenlegungsschrift 2926138 ist eine Einrichtung zur kontinuierlichen Messung des Gehaltes an gelöstem Kohlendioxid in Flüssigkeiten bekannt. Das Meßprinzip beruht auf der Bestimmung der Leitfähigkeitdifferenz. Das Gerät ist mit einer Membram ausgestattet, die auf einer Seite von der gelöstes Kohlendioxid enthaltenden Flüssigkeit, und auf der anderen Seite von einer neutralen oder basischen Meßflüssigkeit angeströmt wird. Je ein Leitfähigkeits-Meßwertaufnehmer ist im Leitungsweg der Meßflüssigkeit vor und nach der permeablen Membran angeordnet. Nachteil der Messung ist, daß sie nicht für eine sich in ihren chemischen und physikalischen Eigenschaften ändernden Flüssigkeiten geeignet ist.

Aus der europäischen Patentanmeldung 0462755 ist es ferner bekannt, Gase, beispielsweise CO₂ durch Infrarot-Absorptionsmessung zu bestimmen. Hierbei wird der Infrarotlichtstrahl durch das zu messende Fluid geschickt. Der Lichtstrahl wird in zwei oder mehr Komponenten geteilt. Diese geteilten Lichtstrahlen werden sodann gemessen. Nachteil dieser Meßanordnung ist, daß sie nicht die Ermittlung von Partialdrücken erlaubt und empfindlich gegenüber streuenden Partikeln der Probenflüssigkeit ist.

Eine Aufteilung in zwei Strahlengängen ist bereits aus der GB 2194333 bekannt. Bei diesem Verfahren wird nur ein Lichtstrahl durch das Meßgut geleitet. Die restliche Strahlung wird als Referenzlicht genutzt, um ebenfalls die Genauigkeit zu erhöhen.

In einer weiteren Veröffentlichung ist ein sog. gechopptes Gasanalysegerät beschrieben, welches ebenfalls mit Lumineszenzdioden arbeitet (Laser und Optoelektronik 17(1985)3, S. 308-310, Wiegleb, G.: Einsatz von LED-Strahlungsquellen in Analysengeräten).

Diesen Geräten und Verfahren ist gemeinsam, daß sie nur Konzentrationen bestimmen. Das Meßgut wird direkt in den Strahlengang gegeben und gemessen. Dies ist möglich für Gase und Flüssigkeiten ohne streuende Partikel mit medienkonstanter Zusammensetzung, in denen Störungen durch einen Blindwert erfaßt werden können. Mit den beschriebenen optischen Methoden lassen sich jedoch nicht Partialdrücke bestimmen. Ebensowenig ist ein Einsatz für medienveränderliche Zusammensetzung und trübende Partikel enthaltende Flüssigkeiten möglich.

Die vorliegende Erfindung hat sich nunmehr die Aufgabe gestellt, eine Vorrichtung zur Messung des Partialdruckes von in Flüssigkeiten gelösten Gasen mittels optischer Methoden zur Verfügung zu stellen, die nicht mehr die geschilderten Nachteile der aus dem Stand der Technik bekannten Vorrichtungen aufweist und die insbesondere die Gaspartialdruckmessung bei längerer Langzeitstabilität der Vorrichtung präzise und in Medien sich ändernder chemisch-physikalischer Zusammensetzung sowie in klaren, trüben und veränderlich trüben Medien zuläßt.

Diese Aufgabe wird dadurch gelöst, daß die Vorrichtung
a) aus einer Meßkammer, die mittels einer gaspermeablen Membrane, die für das zu bestimmende Gas permeabel ist, von einem Probenraum, der die Flüssigkeit mit dem darin gelösten, zu bestimmenden Gas enthält, abgetrennt ist,
b) einer Lichtemissionsquelle zur Erzeugung eines durch die Meßkammer hindurchtretenden Lichtstrahls mit einer Wellenlänge, die durch das zu bestimmende Gas absorbiert wird, und
c) einer Meßanordnung zur Bestimmung des die Meßkammer verlassenden Lichtstrahls besteht, und
d) die Meßkammer mit einem chemisch und biologisch inerten Fluid zur Absorption des in bestimmenden Gases gefüllt ist.

Erfindungsgemäß sind Meßkammer, Lichtemissionsquelle und Meßanordnung in einer stabförmigen Sonde angeordnet. Sofern diese im Bereich der Biotechnologie, z.B. bei Fermentationen, Getränkeherstellungen oder Abwasserreinigungen zum Einsatz kommt, ist sie als sterilisierbare Vorrichtung ausgelegt. Da im Bereich der Fermentationstechnik vorwiegend mittels Dampf sterilisiert wird, sind die Sondenmaterialien auf diese Verhältnisse abzustimmen. Daher kommen die in diesem Bereich bewährten Membranmaterialien auch in erster Linie zum Einsatz. Hierzu zählt vor allem Polytetrafluorethylen (Silicon und andere fluoride Polymere). Bewährt haben sich erfindungsgemäß als gasselektive Membrane Löslichkeitsmembrane. Diese können bei Einsatz in den Probenraum (10) ein Gleichgewicht zwischen der Probenflüssigkeit und dem inneren Gemisch einstellen.

Die Meßkammer ist erfindungsgemäß vorzugsweise mit einem chemisch und biologisch inerten Fluid gefüllt. Dieses wird so ausgewählt, daß es das zu bestimmende Gas, das durch die Membran in die Meßkammer diffundiert, absorbiert. Für diesen Zweck sind in gleicher Weise geeignete Flüssigkeiten oder Gase einsetzbar. Die Art der genannten Fluide richtet sich nach den zu messenden Gasen.

Als Lichtemissionsquelle werden erfindungsgemäß vorzugsweise Lumineszenzdioden eingesetzt. Der Einsatz dieser Vorrichtungen hat folgende Vorteile:

Die Emission ist relativ schmalbandig, d.h. der Einsatz von Interferenzfiltern ist nicht unbedingt erforderlich, um das entsprechende Gas selektiv zu bestimmen. Durch den relativ niedrigen Stromverbrauch ist es prinzipiell möglich, den Meßaufbau tragbar mit Akkubetrieb zu gestalten. Ein entscheidender Vorteil gegenüber den herkömmlichen Infrarotquellen ist die hohe Leistungskonstanz. Daher ist es u.U. möglich, ohne Vergleichsstrecke auszukommen oder Kompensationsschaltungen ohne bewegte Teile aufzubauen. Solch ein System ist mechanisch wenig anfällig. Gleichzeitig garantiert die hohe Leistungskonstanz einen langen Betrieb ohne Nachkalibrierung. Die Lumineszenzdioden sind so klein dimensioniert, daß eine Einkopplung des Lichts in Lichtwellenleiter problemlos möglich ist. So können die sensitiven Teile extern positioniert werden und sind nicht den thermisch-mechanischen Belastungen einer Dampfsterilisation unterworfen.

In dem erfindungsgemäßen Verfahren kann auch mit verschiedenen Wellenlängen, vorzugsweise zwei verschiedenen Wellenlängen, gearbeitet, um die Genauigkeit zu erhöhen. Die Verfahren zur Erhöhung der Meßgenauigkeit und Kompensation von Schwankungen in den elektronischen Bauteilen sind allgemein bekannt und veröffentlicht (Meas.Sci.Technol. 3(1992)2 191-195, Sean F. Johnston: Gas Monitors Employing Infrared LEDs).

Ferner kommen erfindungsgemäß die zu den Lumineszenzdioden kompatiblen Detektoren zum Einsatz. Als solche eignen sich insbesondere Fotodioden, Fotowiderstände und Bleiselenidfotodetektoren (PbSe-Detektoren). Letztere arbeiten vorwiegend im infraroten Bereich und sind vor allem zur Bestimmung von Kohlendioxid geeignet.

Zur Leitung der Lichtwellen von der Lichtemissionsquelle zur Meßkammer werden Lichtwellenleiter eingesetzt. Gleiches gilt für die Leitung des Lichtes von der Meßkammer zur Meßanordnung für die Bestimmung der nicht absorbierten Lichtanteile. Die Meßanordnung ist erfindungsgemäß vorzugsweise mit einer speziellen Schaltung zur Auswertung, Speicherung und Anzeige der Signale verbunden. Aufgrunddessen eignet sich die erfindungsgemäße Vorrichtung insbesondere für die Automatisierung von Anlagen. Mittels einer integrierten Auswertungseinheit können automatisch sämtliche Daten erfaßt und einem Regelungsprozeß zugeführt werden.

Erfindungsgemäß vorteilhaft ist auch die Möglichkeit einer druckfesten Ausgestaltung der Vorrichtung. Es ist lediglich notwendig, die Gehäusekonstruktion der Sonde entsprechend anzupassen. Auf diese Art kann die erfindungsgemäße Vorrichtung bei Drücken von 200 bar eingesetzt werden. Vorzugsweise wird die Sonde bei Drücken bis zu 20 bar verwendet. Bei dem Einsatz für Fermentationsprozesse ist lediglich darauf zu achten, daß die Sonde den unter Sterilisationsbedingungen auftretenden erhöhten Drücken standzuhalten hat.

Weiterer Gegenstand der vorliegenden Erfindung ist ein Verfahren zur Messung des Partialdruckes von in Flüssigkeiten gelösten Gasen. Bei diesem Verfahren wird die erfindungsgemäße Vorrichtung in die in dem Probenraum vorhandene Flüssigkeit derart eingetaucht, daß die Membran vollständig mit Probenflüssigkeit benetzt ist. Infolgedessen kann nunmehr das zu bestimmende Gas selektiv durch die Membran in die Messkammer diffundieren. Durch die Lichtemissionsquelle wird über Lichtwellenleiter ein Lichtstrahl durch die Meßkammer geleitet. Das dort dorthin diffundierende Gas absorbiert einen Teil der Strahlung. Der nicht absorbierte Teil des Lichtstrahls wird über einen Lichtwellenleiter der Meßanordnung für die Bestimmung des Gaspartialdruckes zugeleitet. Durch entsprechende Auswertungs-, Speicherungs- und Anzeigeeinrichtungen läßt sich anhand der Messung des nicht absorbierten Lichtstrahls der Gaspartialdruck bestimmen und auswerten.

Erfindungsgemäß wird vorzugsweise eine durch Lumineszenzdioden erzeugte elektromagnetische Strahlung eingesetzt. Ganz besonders bevorzugt wird der infrarote Bereich.

Die erfindungsgemäße Vorrichtung und das erfindungsgemäße Verfahren eignen sich insbesondere zum Einsatz der Messung des Kohlendioxidpartialdrucks. Kohlendioxid stellt einen beträchtlichen Produktionsfaktor in der Lebensmittelindustrie, insbesondere in der Getränkeindustrie dar. In den Getränken selbst ist Kohlendioxid für die Haltbarkeit und den erfrischenden Geschmack verantwortlich. Die meisten Bestimmungen erfolgen heute über gleichzeitige Druck- und Temperaturkontrolle.

Für eine optimale Prozeßführung biotechnischer Prozesse ist ebenfalls auch eine Kohlendioxidpartialdruckmessung erfordertich. Von Bedeutung in diesem Zusammenhang ist die Tatsache, daß die Versorgung der Mikroorganismen mit Gasen und deren inhibitorischen Eigenschaften eine Funktion der entsprechenden Partialdrücke und nicht der Konzentrationen sind. Trotz dieser Kenntnis wird der Kohlendioxidpartialdruck bis heute nicht hinreichend berücksichtigt. Eine befriedigende Lösung zu seiner Bestimmung ist noch nicht gefunden. Die Hauptprobleme bei der Wahl einer geeigneten Bestimmungsmethode sind fehlende apparative Möglichkeiten und die hohe chemische Stabilität des Kohlendioxids. Kohlendioxid stellt die höchste Oxidationsstufe von Kohlenstoff dar und ist bei Raumtemperatur daher sehr reaktionsträge. In gelöster Form bildet es im Gegensatz zu anderen heterogenen Gasen keine Wasserstoffbrückenbindungen aus. Bei einer Dissoziationskonstante für Kohlensäure von 2x10⁻⁴M liegt nur ein sehr geringer Teil in Form gelöster lonen vor. Eine Meßsonde, die auf der Bestimmung der ionischen Form beruht, ist deshalb schon mit einem Fehler behaftet. Für eine genaue Methode ist es daher erforderlich, direkt das gelöste Kohlendioxid zu bestimmen. Für eine Messung bei Raumtemperaturen steht die Absorptionsmessung von Kohlendioxid zur Verfügung. Die Absorptionsmessung im infratoten Bereich ist mit den vorhandenen Abgasanalysenapparaten Stand der Technik. Die Bestimmung aus der Abluft liefert jedoch Konzentrationen und keine Partialdrücke. Mit Hilfe des Henry'schen Gesetzes lassen sich Konzentrationen in Partialdrücke umrechnen und umgekehrt. Die Umrechnung von Konzentationen in Partialdrücken gestaltet sich für Kohlendioxid im Gegensatz zu Sauerstoff schwieriger, da die Henry-Konstante durch den pH-Wert und Medienbestandteile beeinflußt wird. Fluktuationen des pH-Wertes führen zu zeitlichen Veränderungen der Kohlendioxidkonzentration in der Abluft. Besonders bei basischen Fermentationen und in großen Reaktoren führt die Kohlendioxidspeicherung der Medien zu zeitlichem Überschwingen des Meßsignals bei Annäherung an ein neues Gleichgewicht. Solche Signale können als Änderung des Metabolismus fehlinterpretiert werden.

Durch den Einsatz der erfindungsgemäßen Vorrichtung werden insbesondere die aufgezeigten Probleme der Kohlendioxidpartialdruckmessung gelöst. In diesem Falle wird die Meßkammer mit einem Trägerfluid für Kohlendioxid gefüllt. Dieses Fluid muß eine Löslichkeit für Kohlendioxid aufweisen. Weitere Bedingung ist, daß sie chemisch und biologisch inert ist. Für eine Dampfsterilisation ist weiterhin von Vorteil, wenn das Fluid einen höheren Siedepunkt als das Meßgut besitzt, um Druckschwankungen weitgehend zu vermeiden. Erfindungsgemäß ist die Vorrichtung jedoch nicht auf eine bestimmte Trägerflüssigkeit festgelegt. Deren Zusammensetzung und chemische Natur richten sich vielmehr nach der Art des zu messenden Gases und den Einsatzbedingungen der Sonde.

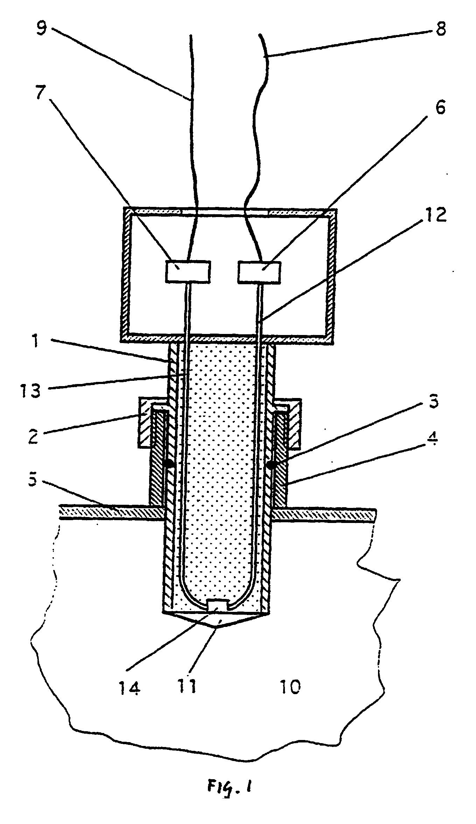

Im folgenden wird die Erfindung unter Bezugnahme auf die Figur näher beschrieben. Die erfindungsgemäße Vorrichtung besteht danach aus der Sonde (1). Der Sondenkörper ist im erfindungsgemäßen Beispiel aus rostfreiem Stahl hergestellt. Es ist jedoch möglich, eine Herstellung aus jedem anderen beliebigen Material vorzunehmen. In der Regel handelt es sich hierbei jedoch um korrosionsfreie Stoffe.

Die Sonde (1) weist ein Anschlußstück (2) auf, welches es erlaubt, die Sonde (1) in die Rohrleitung oder die Wand (5) eines Gefäßes druckfest einzusetzen. Das Anschlußstück (2) und die O-Ring-Anordnung (3) erlauben es, die Sonde (1) abdichtend in einem Zugangsrohr (4) an der Wand (5) zu befestigen. Das Zugangsrohr (4) weist das entsprechende Anschlußstück zum Anschlußstück (2) auf.

Durch diesen Aufbau wird die Möglichkeit gegeben, den Sondenkopf einer Dampfsterilisation zu unterziehen und im Sterilbetrieb zu nutzen.

Innerhalb der Sonde (1) sind eine Lichtquelle (6) und eine Meßanordnung (7) vorhanden. Im erfindungsgemäßen Beispiel handelt es sich bei der Lichtquelle (6) um eine Lumineszenzdiode und bei der Meßanordnung (7) um einen Fotoempfänger. Beide Geräteteile sind mit den elektrischen Leitungen (8) und (9) versehen. Die Lumineszenzdiode (6) wird über die Leitung (8) mit Strom versorgt. Der Fotoempfänger (7) überträgt einen Signalimpuls über die Leitung (9) zu einem Mittel zum Verstärken und Aufzeichnen des Signals.

Die Lumineszenzdiode (6) und der Fotoempfänger (7) sind außerhalb des Flüssigkeitsraums (10) angeordnet. Sie sind über die extrinsischen Lichtwellenleiter (12) und (13), welche zum Übertragen des Lichts (12) von der Limineszenzdiode (6) und des nicht absorbierten Lichts zum Fotoempfänger (7) dienen, eingesetzt. Die Lichtwellenleiter können aus beliebigen für die Übertragung von Licht geeigneten Materialien hergestellt sein. Im erfindungsgemäßen Beispiel wird im infraroten Bereich gearbeitet. Es kommen daher vorzugsweise Lichtleiter aus transparentem Material, z.B. aus Silberhalogeniden und Chalcogeniden in Betracht.

Diese Lichtwellenleiter sind thermisch belastbar und eignen sich somit für den Einsatz in dampfsterilisierbarer Umgebung.

An der Spitze des Kopfes der Sonde (1) befindet sich der Meßraum (14). Dieser ist im erfindungsgemäßen Beispiel mit einem chemisch und biologisch inerten Fluid gefüllt, das ein hohes physikalisches Absorptionsvermögen für Kohlendioxid aufweist. In Fermentationsverfahren werden Fluide ausgewählt, deren Siedepunkt derart gewählt ist, daß es während der Sterilisation nicht zu Druckschwankungen kommt.

Der Meßraum (14) ist von dem Probenraum (10) über die gaspermeable Membran (11) getrennt. Die Membran (11) ist im erfindungsgemäßen Beispiel eine thermisch stabile Membran, die aus dampfsterilisierbarem Material gefertigt ist. Erfindungsgemäß wird hierfür Polytetrafluorethylen und/oder Silicon bevorzugt.

Das gelöste Gas diffundiert durch die Membran (11) in den Probenraum (10) bis zur Einstellung eines Gleichgewichtes. Da die Diffusion von Gasen durch eine Membran partialdruckkontrolliert ist, bestimmt die Sonde (1) den Partialdruck. Damit mißt die Sonde einen biologisch bedeutsamen Parameter; denn die Versorgung der Microorganismen ist, wie alle Transportvorgänge aus den Zellen bzw. in die Zellen, partialdruck- und nicht konzentrationskontrolliert.

Die Lumineszenzdiode (6) emittiert schmalbandiges Licht, das selektiv durch das zu bestimmende Gas absorbiert wird. Die Wellenlänge kann in bezug auf das zu untersuchende Gas sowohl im UV/VIS als auch im Infrarotbereich liegen. Für Kohlendioxid sind dies vorzugsweise 4,3 µm.

Der emittierte Wellenlängenbereich kann beschränkt sein durch einen Wärmenstrahler mit Interferenzfilter oder vorzugsweise durch eine schmalbandige Lumineszenzdiode. Der besondere Vorteil beim Einsatz der Luminessenzdiode ist, daß die Strahlung moduliert werden kann, was die Detektion erhöht und Effekte wie Gleichstromdrift minimiert.

Die emittierte Strahlung wird über den Lichtwellenleiter (12) dem Meßraum zugeführt. Das vorhandene Gas schwächt spezifisch die emittierte Strahlung ab. Das abgeschwächte Licht wird zum Teil vom Lichtwellenleiter (13) aufgenommen und dem Fotoempfänger (7) zugeführt. Dieser mißt das abgeschwächte Licht und produziert ein elektrisches Signal proportional zum abgeschwächten Licht. Wenn moduliertes Licht eingesetzt wird, läßt sich das elektrische Signal ebenfalls modulieren.

Die Länge der Meßkammer (14) entspricht der optischen Weglänge. Eine optimale optische Weglänge wird im Meßraum (14) so gewählt, daß die Sonde (1) den gesamten Meßbereich erfaßt. Der Meßbereich ist umgekehrt proportional zur Weglänge. Je kleiner somit die Weglänge der Meßkammer (14) der Sonde (1) ist, desto größer ist der detektierbare Bereich und desto kleiner ist die Auflösung.

Die mit der Erfindung erzielten Vorteile bestehen insbesondere darin, daß vor allem im Fall der Kohlendioxidpartialdruckmessung durch Trennung des Meßraumes vom Probenraum nicht durch die Anwesenheit von trübenden und sich in ihrer Konzentration ändernden Partikeln Einflüsse entstehen. Des weiteren wird durch die Implementierung der Membran die Messung des Partialdrucks garantiert. Es ist zwar prinzipiell möglich, mit Hilfe des Henry'schen Gesetzes Konzentration in Partialdrücke umzurechnen. Doch erfordert es die gleichzeitige Kenntnis von Temperatur und Druck sowie der Medieneigenschaften. Letzteres ist insbesondere bei der Anwendung von Fermentationsmedien schwierig. Weiterhin werden die Langzeitstabilität, Genauigkeit und der Meßbereich gegenüber pHsensitiven Partialdrucksonden erhöht.

Die erfindungsgemäße Sonde ist sowohl in der Getränkeindustrie als auch in der Biotechnologie besonders gut einsetzbar. Für den Einsatz in der Lebensmitteltechnologie lassen sich Sonden für Meßbereiche von bis zu 10 bar erstellen.

Beim Einsatz für die Kohlendioxidpartialdruckmessung im Bereich der Fermentationstechnik ist von Vorteil, daß eine Vorkalibrierung möglich ist. Denn aufgrund des inhibierenden Einflusses von Kohlendioxid auf die meisten Organismen kann eine Nachkalibrierung nicht mehr vorgenommen werden. Vorteilhaft in diesem Anwendungsbereich ist darüber hinaus, daß die Sonde während der Sterilisation thermischen Belastungen standhält und Temperaturen von 150°C ohne weiteres zu widerstehen vermag. Schließlich ist von Vorteil, daß im Gegensatz zu den bisherigen Verfahren mit Absorptionsmessung eine Störung durch Stoffe, die ebenfalls im infraroten Bereich absorbieren, ausgeschlossen ist.

## Patentansprüche

1. Vorrichtung zur Messung des Partialdrucks von in Flüssigkeiten gelösten Gasen, enthaltend
a) eine Meßkammer (14), die mittels einer gaspermeablen Membrane (11), die für das zu bestimmende Gas permeabel ist, von einem Probenraum (19), der die Flüssigkeit mit dem darin gelösten, zu bestimmenden Gas enthält, abgetrennt ist,
b) eine Lichtemissionsquelle (6) zur Erzeugung eines durch die Meßkammer (14) hindurchtretenden Lichtstrahls mit einer Wellenlänge, die durch das zu bestimmende Gas absorbiert wird,
c) eine Meßanordnung (7) zur Bestimmung des die Meßkammer (14) verlassenden Lichtstrahls,
**dadurch gekennzeichnet,daß**
d) die Meßkammer (14) mit einem chemisch und biologisch inerten Fluid zur Absorption des zu bestimmenden Gases gefüllt ist.

2. Vorrichtung nach Anspruch 1,
**dadurch gekennzeichnet, daß** die Meßkammer (14), die Lichtemissionsquelle (6), und die Meßanordnung (7) in einer stabförmigen Sonde (1) angeordnet sind.

3. Vorrichtung nach Anspruch 2,
**dadurch gekennzeichnet, daß** die Sonde (1) sterilisierbar ist.

4. Vorrichtung nach Anspruch 3,
**dadurch gekennzeichnet, daß** die Sonde (1) mittels Dampf sterilisierbar ist.

5. Vorrichtung nach Anspruch 1 bis 4,
**dadurch gekennzeichnet, daß** die Membranaus Polytetrafluorathylen besteht.

6. Vorrichtung nach Anspruch 1 bis 5,
**dadurch gekennzeichnet, daß** die Membran eine gasselektive Löslichkeitsmembran ist, über die sich ein Gleichgewicht zwischen Probenraum (10) und Meßkammer (14) einstellt.

7. Vorrichtung nach einem der Ansprüche 1 bis 6,
**dadurch gekennzeichnet, daß** das Fluid eine Flüssigkeit ist.

8. Vorrichtung nach einem der Ansprüche 1 bis 6,
**dadurch gekennzeichnet, daß** das Fluid ein Gas ist.

9. Vorrichtung nach Anspruch 1 bis 7,
**dadurch gekennzeichnet, daß** in ihr ein Lichtwellenleiter (12) zur Leitung des Lichtstrahls von der Lichtemissionsquelle (6) zur Meßkammer (14) und ein Lichtwellenleiter (13) zur Leitung des Lichts von der Meßkammer (14) zur Meßanordnung (7) angeordnet ist.

10. Vorrichtung nach Anspruch 1 bis 9,
**dadurch gekennzeichnet, daß** die Lichtemissionsquelle (6) eine Lumineszenzdiode ist.

11. Vorrichtung nach Anspruch 1 bis 10,
**dadurch gekennzeichnet, daß** die Meßanordnung (7) eine Fotodiode, ein Fotowiderstand oder ein Bleiselenidfotodetektor ist.

12. Vorrichtung nach Anspruch 1 bis 11,
**dadurch gekennzeichnet, daß** die Meßanordnung (7) mit einer Schaltungsanordnung zur Auswertung, Speicherung und Anzeige der Signale verbunden ist.

13. Vorrichtung nach Anspruch 1 bis 12,
**dadurch gekennzeichnet, daß** sie druckfest ausgestaltet ist.

14. Vorrichtung nach Anspruch 13,
**dadurch gekennzeichnet, daß** für den Betrieb unter Drücken bis zu 200 bar, vorzugsweise bis zu 20 bar ausgelegt ist.

15. Verfahren zur Messung des Partialdruckes von in Flüssigkeiten gelösten Gasen mit einer Vorrichtung gem. einem der Ansprüche 1 bis 14,
**dadurch gekennzeichnet, daß**
a) deren Membran (11) in die in dem Probenraum (10) vorhandene Flüssigkeit eingetaucht wird,
b) das in der Flüssigkeit vorhandene, zu bestimmende Gas in die Meßkammer (14) durch die Membran (11) diffundiert,
c) ein Lichtstrahl mit einer Wellenlänge, die von dem zu bestimmenden Gas absorbiert wird, durch die Meßkammer (14) geleitet wird, und
d) das nicht absorbierte Licht der Meßanordnung (7) zugeleitet wird.

16. Verfahren nach Anspruch 15,
**dadurch gekennzeichnet, daß** die Messung mittels Infrarotstrahlung durchgeführt wird.

17. Verwendung der Vorrichtung nach einem der Ansprüche 1 bis 14, zur Bestimmung des Partialdruckes von Sauerstoff oder Kohlendioxid.

18. Verwendung der Vorrichtung nach einem der Ansprüche 1 bis 14, zur Messung, Steuerung und Regelung von Fermentationsprozessen, Getränkeherstellungsverfahren und Abwasserreinigungsanlagen.

## Revendications

1. Dispositif de mesure de la pression partielle de gaz dissous dans des liquides, contenant
a) une chambre de mesure (14), qui est séparée, à l'aide d'une membrane perméable aux gaz (11), qui est perméable au gaz à déterminer, d'un espace échantillon (19), qui contient le liquide avec le gaz à déterminer qui y est dissous,
b) une source d'émission lumineuse (6) en vue de la production d'un rayon lumineux traversant la chambre de mesure (14) d'une longueur d'onde, qui est absorbée par le gaz à déterminer,
c) un arrangement de mesure (7) en vue de la détermination du rayon lumineux quittant la chambre de mesure (14),
**caractérisé en ce que**
d) la chambre de mesure (14) est remplie d'un fluide inerte du point de vue chimique et biologique en vue de l'absorption du gaz à déterminer.

2. Dispositif selon la revendication 1, **caractérisé en ce que** la chambre de mesure (14), la source d'émission lumineuse (6) et l'arrangement de mesure (7) sont disposés dans une sonde en forme de tige (1).

3. Dispositif selon la revendication 2, **caractérisé en ce que** la sonde (1) peut être stérilisée.

4. Dispositif selon la revendication 3, **caractérisé en ce que** la sonde (1) peut être stérilisée à l'aide de vapeur.

5. Dispositif selon la revendication 1 à 4, **caractérisé en ce que** la membrane se compose de polytétrafluoroéthylène.

6. Dispositif selon les revendications 1 à 5, **caractérisé en ce que** la membrane est une membrane de solubilité à sélectivité vis-à-vis des gaz, par l'intermédiaire de laquelle s'établit un équilibre entre l'espace échantillon (10) et la chambre de mesure (14).

7. Dispositif selon l'une quelconque des revendications 1 à 6, **caractérisé en ce que** le fluide est un liquide.

8. Dispositif selon l'une quelconque des revendications 1 à 6, **caractérisé en ce que** le fluide est un gaz.

9. Dispositif selon les revendications 1 à 7, **caractérisé en ce que** l'on y arrange un guide d'ondes lumineuses (12) en vue de la conduite du rayon lumineux de la source d'émission lumineuse (6) vers la chambre de mesure (14) et un guide d'ondes lumineuses (13) en vue de la conduite de la lumière de la chambre de mesure (14) vers l'arrangement de mesure (7).

10. Dispositif selon les revendications 1 à 9, **caractérisé en ce que** la source d'émission lumineuse (6) est une diode luminescente.

11. Dispositif selon les revendications 1 à 10, **caractérisé en ce que** l'arrangement de mesure (7) est une photodiode, une photorésistance ou un photodétecteur au séléniure de plomb.

12. Dispositif selon les revendications 1 à 11, **caractérisé en ce que** l'arrangement de mesure (7) est relié à un arrangement de commutation en vue de l'évaluation, de la mise en mémoire et de l'affichage des signaux.

13. Dispositif selon les revendications 1 à 12, **caractérisé en ce qu'**il est équipé pour résister à la pression.

14. Dispositif selon la revendication 13, **caractérisé en ce qu'**il est conçu pour le fonctionnement à des pressions allant jusqu'à 200 bars, de préférence, jusqu'à 20 bars.

15. Procédé de mesure de la pression partielle de gaz dissous dans des liquides à l'aide d'un dispositif selon l'une quelconque des revendications 1 à 14, **caractérisé en ce que**
a) sa membrane (11) est immergée dans le liquide présent dans l'espace échantillon (10),
b) le gaz à déterminer, présent dans le liquide, diffuse à travers la membrane (11) dans la chambre de mesure (14),
c) un rayon lumineux ayant une longueur d'onde, qui est absorbée par le gaz à déterminer, est conduit à travers la chambre de mesure (14), et
d) la lumière non absorbée est acheminée à l'arrangement de mesure (7).

16. Procédé selon la revendication 15, **caractérisé en ce que** la mesure est effectuée à l'aide de rayonnement infrarouge.

17. Utilisation du dispositif selon l'une quelconque des revendications 1 à 14, en vue de la détermination de la pression partielle d'oxygène ou de dioxyde de carbone.

18. Utilisation du dispositif selon l'une quelconque des revendications 1 à 14, en vue de la mesure, de la commande et de la régulation de processus de fermentation, de procédés de fabrication de boissons et d'installations de purification d'eaux résiduaires.

## Claims

1. Device for measuring the partial pressure of gases dissolved in liquids, comprising
a) a measuring chamber (14) which is separated, by means of a gas-permeable membrane (11) which is permeable to the gas to be determined, from a sample space (19) [sic] which contains the liquid and, dissolved therein, the gas to be determined,
b) a light-emission source (6) for generating a light beam which passes through the measuring chamber (14) and has a wavelength which is absorbed by the gas to be determined,
c) a measuring arrangement (7) for determining the light beam emerging from the measuring chamber (14),
**characterized in that**
d) the measuring chamber (14) is filled with a chemically and biologically inert fluid for absorbing the gas to be determined.

2. Device according to Claim 1, **characterized in that** the measuring chamber (14), the light-emission source (6) and the measuring arrangement (7) are arranged in a rod-shaped probe (1).

3. Device according to Claim 2, **characterized in that** the probe (1) is sterilizable.

4. Device according to Claim 3, **characterized in that** the probe (1) can be sterilized using steam.

5. Device according to Claims 1 to 4, **characterized in that** the membrane consists of polytetrafluoroethylene.

6. Device according to Claims 1 to 5, **characterized in that** the membrane is a gas-selective solubility membrane, through which equilibrium is established between the sample space (10) and the measuring chamber (14).

7. Device according to one of Claims 1 to 6, **characterized in that** the fluid is a liquid.

8. Device according to one of Claims 1 to 6, **characterized in that** the fluid is a gas.

9. Device according to Claims 1 to 7, **characterized in that** it contains an optical waveguide (12) for guiding the light beam from the light-emission source (6) to the measuring chamber (14) and an optical waveguide (13) for guiding the light from the measuring chamber (14) to the measuring arrangement (7).

10. Device according to Claims 1 to 9, **characterized in that** the light-emission source (6) is a light-emitting diode.

11. Device according to Claims 1 to 10, **characterized in that** the measuring arrangement (7) is a photodiode, a photoresistor or a lead selenide photodetector.

12. Device according to Claims 1 to 11, **characterized in that** the measuring arrangement (7) is connected to a circuit arrangement for evaluating, storing and displaying the signals.

13. Device according to Claims 1 to 12, **characterized in that** it is of pressure-proof design.

14. Device according to Claim 13, **characterized in that** [lacuna] is designed for operation under pressures of up to 200 bar, preferably up to 20 bar.

15. Method for measuring the partial pressure of gases dissolved in liquids using a device according to one of Claims 1 to 14, **characterized in that**
a) the membrane (11) of this device is immersed in the liquid present in the sample space (10),
b) the gas which is present in the liquid and is to be determined diffuses into the measuring chamber (14) through the membrane (11),
c) a light beam having a wavelength which is absorbed by the gas to be determined is guided through the measuring chamber (14), and
d) the unabsorbed light is fed to the measuring arrangement (7).

16. Method according to Claim 15, **characterized in that** the measurement is carried out using infrared radiation.

17. Use of the device according to one of Claims 1 to 14 for determining the partial pressure of oxygen or carbon dioxide.

18. Use of the device according to one of Claims 1 to 14 for measuring, controlling and regulating fermentation processes, methods for the production of drinks, and waste-water purification plants.
